# EUROPÄISCHE PATENTSCHRIFT

(11) **EP 0 969 510 B1**
(45) Veröffentlichungstag und Bekanntmachung des Hinweises auf die Patenterteilung: **17.04.2002**
(21) Anmeldenummer: 99112071.8
(22) Anmeldetag: 23.06.1999
(51) Int. Cl.: H01L 25/065, H01L 25/07, G01N 27/403, G01N 27/414, G01N 27/28, G01N 33/487

(54) **Chip-Anordnung**
Chip arrangement
Agencement d'une puce électronique

(30) Priorität: 30.06.1998 DE 19829121
(43) Veröffentlichungstag der Anmeldung: 05.01.2000
(60) Teilanmeldung: 01126916.4
(73) Patentinhaber: Micronas GmbH, 79108 Freiburg (DE)
(72) Erfinder: Sieben, Ulrich, Dr.Dipl.Phys., 79276 Reute (DE); Igel, Günter, Dipl-Ing., 79331 Teningen (DE); Lehmann, Mirko, Dipl.Phys., 79117 Freiburg (DE); Gahle, Hans-Jürgen, Dr., 79312 Emmendingen (DE); Wolf, Bernhard, Prof.Dr., 79252 Stegen (DE); Baumann, Werner, Dr., 79199 Freiburg (DE); Ehret, Ralf, Dr., 79291 Merdingen (DE)
(74) Vertreter: Bickel, Michael

(56) Entgegenhaltungen:
- EP-A- 0 190 005
- EP-A- 0 399 227
- WO-A-87/05747
- WO-A-97/42478
- DE-A- 2 736 200
- US-A- 5 114 859

## Beschreibung

Die Erfindung betrifft eine Chip-Anordnung nach dem Oberbegriff von Anspruch 1.

Eine solche Chip-Anordnung ist aus EP 190 005 A bekannt. Sie weist eine Substratplatte auf, die einen Durchbruch hat, in den ein etwa rechteckiger Silizium-Trägerchip derart eingesetzt ist, daß er mit seinen Enden die einander abgewandten flachseitigen Oberflächen der Substratplatte überragt und dadurch Überstände bildet. Dabei ist die Ebene des Trägerchips rechtwinklig zur Ebene der Substratplatte angeordnet. An dem die eine flachseitige Oberfläche überragenden Überstand sind zwei als chemischen Sensoren ausgebildete Bauelemente und an dem die andere Oberfläche überragenden Überstand Anschlußkontakte angeordnet. Diese sind über Leiterbahnen, die an der Oberfläche des Trägerchips verlaufen und den Durchbruch der Substratplatte durchsetzen, über eine aktive elektronische Schaltung mit den Sensoren verbunden. Zwischen der Substratplatte und dem Trägerchip ist eine Abdichtung vorgesehen, die bewirkt, daß nur die Sensoren mit der zu analysierenden Flüssigkeit in Berührung kommen, während die Anschlußkontakte durch die Substratplatte vor dieser Flüssigkeit geschützt sind. Die vorbekannte Chip-Anordnung ist jedoch zu Untersuchung von Flüssigkeiten, die unterschiedlich große Partikel enthalten, von denen Partikel, die eine vorgegebene Größe überschreiten von den Sensoren ferngehalten werden sollen, nicht geeignet.

Es besteht deshalb die Aufgabe, eine Chip-Anordnung nach dem Oberbegriff von Patentanspruch 1 zu schaffen, bei der Partikel, die eine vorgegebene Größe überschreiten von dem Bauelement ferngehalten werden und die einfach und kostengünstig herstellbar ist.

Diese Aufgabe wird mit den Merkmalen des Patentanspruchs 1 gelöst.

Das elektrische oder elektronische Bauelement ist dabei in einem durch den Trägerchip und die Substratplatte begrenzten Eckbereich angeordnet, so daß nur Partikel, die eine durch die Abmessungen des Eckbereichs vorgegebene Größe nicht überschreiten, mit dem elektrischen oder elektronischen Bauelement in Kontakt geraten können. Somit ergibt sich ein einfach aufgebauter mechanischer Filter, der das Vordringen größerer Partikel zu dem Bauelement verhindert. Der Anschlußkontakt befindet sich an der dem elektrischen oder elektronischen Bauelement abgewandten Rückseite der Substratplatte, so daß die im Bereich des das Bauelement aufweisenden Überstands befindlichen Leiterbahnbereiche vollständig mit einer Passivierungsschicht abgedeckt werden können. Eine solche Passivierungsschicht kann beispielsweise in Dünnschichttechnologie mit großer Genauigkeit und Feuchtigkeitsfestigkeit hergestellt werden, so daß eine Korrosion an der in den Trägerchip integrierten Leiterbahn durch das mit dem elektrischen oder elektronischen Bauelement zu untersuchenden oder zu behandelnden Medium weitestgehend vermieden wird. Die zwischen dem Trägerchip und der Substratplatte angeordnete Abdichtung verhindert, daß das an der Vorderseite der Substratplatte befindliche Medium zu dem an der Rückseite der Substratplatte angeordneten Anschlußkontakt gelangen kann. Der in der Substratplatte angeordnete Durchbruch kann beispielsweise mittels Ultraschallbohren in die Substratplatte eingebracht werden. Die Chip-Anordnung ist somit einfach und kostengünstig herstellbar.

Zweckmäßigerweise ist der Trägerchip lösbar mit der Substratplatte verbindbar ist. Der Trägerchip kann dann gegebenenfalls leicht ausgetauscht werden, wenn das Bauelement seine vorgesehene Lebensdauer erreicht hat oder wenn es durch einen Kontakt mit einem zu untersuchenden oder zu behandelnden, chemisch agressiven Medium, einmal ausfallen sollte.

Bei einer bevorzugten und besonders vorteilhaften Ausführungsform der Erfindung ist der Trägerchip mit einem den Durchbruch begrenzenden Wandungsbereich der Substratplatte verklebt. Der zwischen dem Trägerchip und der Substratplatte angeordnete Klebstoff dient dann einerseits dazu, den Trägerchip an der Substratplatte zu fixieren und dichtet andererseits aber auch den Durchbruch der Substratplatte gegen den Trägerchip ab, so daß ein an der Vorderseite der Substratplatte im Bereich des elektrischen oder elektronischen Bauelements befindliches Medium nicht an die den Anschlußkontakt aufweisende Rückseite der Substratplatte gelangen kann. Der Klebstoff gleicht außerdem Toleranzen in den Abmessungen des Trägerchips und/oder dem in der Substratplatte befindlichen Wandungsdurchbruch, in den der Trägerchip eingesetzt ist, aus. Die Chip-Anordnung ist dadurch noch einfacher und kostengünstiger herstellbar.

Eine bevorzugte Ausführungsform der Erfindung sieht vor, daß sich der Querschnitt des das elektrische oder elektronische Bauelement aufweisenden Überstandes ausgehend von der Oberfläche der Substratplatte zu der am weitesten vorstehenden Stelle des Überstandes verjüngt. Der das Bauelement aufweisende Überstand weist also eine Spitze auf. Bei einer Chip-Anordnung, bei der das elektronische Bauelement ein Sensor ist, kann die Substratplatte mit ihrer Flachseite beispielsweise auf eine zu untersuchende Hautschicht aufgelegt werden, wobei der den Sensor aufweisende spitze Überstand mit einer der Höhe des Überstands entsprechenden definierten Tiefe in die Hautschicht eindringt, so daß dort Meßwerte entnommen werden können. So können zum Beispiel die GlucoseKonzentration, die Feuchtigkeit der Haut, eine Ionenkonzentration, ein Gasgehalt oder dergleichen physiologische Parameter gemessen werden, die Aussagen über die Vitalität der Haut und/oder des dahinter befindlichen Gewebebereiches ermöglichen. Dabei ist es sogar möglich, daß die an der Hautschicht anliegende Substratplatte parallel zur Oberfläche der Hautschicht verschoben wird, so daß der den Sensor aufweisende Überstand parallel zur Oberfläche der Hautschicht durch diese hindurch gezogen wird. Dadurch kann auf einfache Weise entlang einer parallel zur Oberfläche der Hautschicht verlaufenden Linie ein Meßprofil erstellt werden. Selbstverständlich kann die den spitzen Überstand aufweisende Chip-Anordnung aber auch zum Untersuchen oder Behandeln anderer weicher Körper verwendet werden, in die der das elektrische oder elektronische Bauelement aufweisende spitze Vorsprung beim Andrücken der Substratplatte an den Körper eindringen kann.

Eine vorteilhafte Ausführungsform der Erfindung sieht vor, daß der Trägerchip bei der Montage der Chip-Anordnung in wenigstens zwei unterschiedlichen Lagen in den Durchbruch der Substratplatte einsetzbar ist, daß in einer dieser Lagen wenigstens ein elektrisches oder elektronisches Bauelement an einem eine flachseitige Oberfläche der Substratplatte überragenden Überstand des Trägerchips und der (die) diesem (diesen) Bauelement(en) zugeordnete(n) Anschlußkontakt(e) an dem die andere flachseitige Oberfläche der Substratplatte überragenden Überstand angeordnet ist, und daß in der anderen Lage des Trägerchips das (die) Bauelement(e) und der (die) .Anschlußkontakt(e) an demselben, eine flachseitige Oberfläche der Substratplatte überragenden Überstand des Trägerchips angeordnet sind. Dadurch ist es möglich, das Baulelement durch entsprechendes Einsetzen des Trägerchips in die Substratplatte nur für die Dauer einer Messung oder einer Behandlung mit einem an einer flachseitigen Oberflächen der Substratplatte befindlichen Objekt, beispielsweise einem chemisch agressiven Medium, in Berührung zu bringen, während das Baulelement außerhalb der Meß- oder Behandlungsphase an der dem Objekt abgewandten flachseitigen Oberflächen der Substratplatte angeordnet ist. Das Bauelement kommt also nur vorübergehend mit dem agressiven Medium in Verbindung, wodurch sich seine Lebensdauer entsprechend verlängert.

Bei einer besonders vorteilhaften Weiterbildung der Erfindung ist vorgesehen, daß der Trägerchip bei der Montage der Chip-Anordnung in wenigstens zwei unterschiedlichen Lagen in den Durchbruch der Substratplatte einsetzbar ist, daß der Trägerchip wenigstens zwei elektrische oder elektronische Bauelemente aufweist, die jeweils mittels wenigstens einer Leiterbahn mit -zumindest einem ihnen jeweils zugeordneten elektrischen Anschlußkontakt verbunden sind, und daß je nach gewählter Lage des Trägerchips jeweils wenigstens eines dieser Bauelemente an einem eine flachseitige Oberfläche der Substratplatte überragenden Überstand des Trägerchips und der (die) diesem (diesen) Bauelement(en) zugeordnete(n) Anschlußkontakt an dem die andere flachseitige Oberfläche der Substratplatte überragenden Überstand angeordnet ist. Dadurch ist je nach gewählter Lage des Trägerchips ein anderes Bauelement oder sogar mehrere andere Bauelemente an dem in Gebrauchsstellung dem zu untersuchenden oder zu behandelnden Objekt zugewandten Überstand des Trägerchips angeordnet. Bei einem Trägerchip mit mehreren gleichen Bauelementen verlängert sich dadurch die Lebensdauer der Chip-Anordnung entsprechend, da ein Bauelement, das beispielsweise durch einen längeren Kontakt mit einem chemisch agressiven Medium unbrauchbar geworden ist, durch entsprechendes Umsetzen des Trägerchips auf einfache Weise durch ein anderes, funktionsfähiges Bauelement ersetzt werden kann. Der Trägerchip kann aber auch voneinander verschiedene Bauelemente aufweisen. Dadurch ergibt sich ein Bausatz zum Erstellen einer Chip-Anordnung, mit dem je nach gewählter Lage des Trägerchips in dem Durchbruch der Substratplatte unterschiedliche Chip-Anordnungen hergestellt werden können. Die elektrischen oder elektronischen. Bauelemente können beispielsweise am Umfang des Trägerchips verteilt in dessen flachseitige Oberfläche integriert sein, wobei der Trägerchip in unterschiedlichen Drehlagen in Bezug zu der Normalen auf diese Oberfläche in die Substratplatte einsetzbar ist. Abhängig von der jeweiligen Drehlage des Trägerchips sind dann jeweils andere Bauelemente oder Sensoren an der Vorderseite der Substratplatte angeordnet, während die diesen zugeordneten. Anschlußkontakte sich jeweils an der Rückseite der Substratplatte befinden.

Vorteilhaft ist, wenn auf dem elektrischen oder elektronischen Bauelement eine ionendurchlässige Membran angeordnet ist. Dadurch können Ionen bis an das Bauelement beziehungsweise den Sensor gelangen, während andere Substanzen durch die Membran von dem Sensor ferngehalten werden. Dabei ist es sogar möglich, daß die Membran nur für bestimmte Ionen durchlässig ist, so daß deren Konzentration in einem zu untersuchenden Medium selektiv gemessen werden kann. Zweckmäßigerweise wird die Membran nach dem Einsetzen des Trägerchips in die Substratplatte auf das elektrische oder elektronische Bauelement aufgetragen. Dazu wird das Membranmaterial zunächst in einer flüchtigen Flüssigkeit, beispielsweise in Alkohol oder Aceton gelöst. Die Chip-Anordnung wird so ausgerichtet, daß die das Bauelement aufweisende Oberfläche des Trägerchips schräg zur Horizontalen, insbesondere vertikal verläuft. Dann wird auf die benachbart zu dem Bauelement angeordnete, quer zu der das Bauelement aufweisenden Oberfläche des Trägerchips verlaufende stirnseitige Randfläche des Trägerchips eine geringe Menge der das Membranmaterial enthaltenden Flüssigkeit aufgetragen, derart, daß ein Teil dieser Flüssigkeit schwerkraftbedingt von der stirnseitigen Randfläche des Trägerchips über das an der quer dazu angeordneten Oberfläche des Trägerchips befindliche elektrische oder- elektronische Bauelement fließt, so daß sich auf diesem eine dünne Flüssigkeitsschicht bildet. Nach dem Verdunsten dieser Flüssigkeitsschicht verbleibt dann auf dem elektrischen oder elektronischen Bauelement eine Membranschicht, die auf dem Bauelement eine gleichmäßige Dicke aufweist.

Bei einer vorteilhaften Ausführungsform der Erfindung weist die Substratplatte im Bereich des Meß- oder Wirkraumes des elektrischen oder elektronischen Bauelements wenigstens einen Vorsprung auf, der zusammen mit dem das Bauelement aufweisenden Überstand einen mechanischen Filter bildet. Dabei wird unter einem Wirkraum bei einem eine elektromagnetische Strahlung aussendenden Bauelement der Raum verstanden, in den das Bauelement die Strahlung aussendet Entsprechend wird bei einem Bauelement, von dem ein elektrisches und/oder magnetisches Feld ausgeht, der Raum verstanden, in dem dieses Feld wirksam ist. Der mechanische Filter weist also einen mit dem das Bauelement aufweisenden Überstand zusammenwirkenden Vorsprung auf, wobei zwischen dem Bauelement und dem Vorsprung ein Freiraum angeordnet ist, der den Zugang zu dem Bauelement bildet. Dadurch werden Partikel, deren Abmessungen größer sind als diejenigen des Freiraums von dem Meß- oder Wirkraum des Bauelements ferngehalten, während kleinere Partikel in den Meß- oder Wirkraum und gegebenenfalls bis an das Bauelement selbst gelangen können. Der Vorsprung kann auch ein an der Substratplatte befindlicher Absatz oder eine Stufe sein.

Eine Ausführungsform sieht vor, daß der Vorsprung des mechanischen Filters durch den Überstand eines in einen Durchbruch der Substratplatte eingesetzten Plättchens gebildet ist. Der den Vorsprung kann dann bei der Herstellung der Chip-Anordnung in gleicher Weise an der Substratplatte angebracht werden, wie der das elektrische oder elektronische Bauelement aufweisende Überstand des Trägerchips. Die Chip-Anordnung ist dadurch noch einfacher herstellbar. Gegebenenfalls kann der Vorsprung des mechanischen Filters auch durch den Überstand eines weiteren Trägerchips gebildet sein.

Vorteilhaft ist, wenn die das elektrische oder elektronische Bauelement aufweisende Trägerchip-Oberfläche und die dieser zugewandte Oberfläche des im Bereich des Meß- oder Wirkraums des Bauelements angeordneten Vorsprungs in der Oberflächenebene der Substratplatte trichterförmig schräg zueinander verlaufen. Dadurch ergibt sich ein trichterförmiger Kanal, der für ein an der Substratplatte befindliches Medium einen strömungsrichtungsabhängigen Filter bildet.

Zum Filtern kleiner Partikel, beispielsweise solcher mit einem Durchmesser, der kleiner als 1 um ist, ist es vorteilhaft, wenn an dem Trägerchip ein Körper anliegt, der das elektrische oder elektronische Bauelement überdeckt, daß als Abstandshalter an dem Trägerchip mindestens ein seitlich über die Oberflächenebene des Bauelements vorstehender, an dem Körper anliegender Bereich und/oder an dem Körper ein seitlich über den das Bauelement überdeckenden Oberflächenbereich vorstehender, an dem Trägerchip anliegender Bereich angeordnet ist, derart, daß zwischen dem Bauelement und dem Körper ein den Zugang zu dem Bauelement bildender Freiraum oder Spalt angeordnet ist. Dadurch ist der Körper bei der Montage der Chip-Anordnung einfacher und mit größerer Genauigkeit an dem Trägerchip positionierbar. Der gegenüber dem Bauelement vorstehende Bereich kann mit bekannten Verfahren der Halbleitertechnik, beispielsweis in Maskentechnik mit großer Maßgenauigkeit hergestellt werden, was insbesondere die Realisierung kleiner Spaltmaße bzw. Freiräume zwischen dem Bauelement und dem Körper mit eng tolerierten Abmessungen ermöglicht. Der an dem vorstehende Trägerchip-Bereich anliegende Körper kann einen im wesentlichen ebenen, dem Bauelement zugewandten, parallel zu dessen Oberflächenebene angeordneten und vorzugsweise an dem vorstehenden Trägerchip-Bereich anliegenden Oberflächenbereich aufweisen. Der Körper kann beispielsweise ein zweiter Trägerchip sein, der an dem vorstehenden Bereich des ersten Trägerchips plan anliegt. Die Herstellung des gegenüber dem Bauelement vorstehenden Trägerchip-Bereichs kann beispielsweise in der Weise erfolgen, daß in die Oberfläche des Trägerchips eine Vertiefung eingeätzt wird, in welcher das Bauelement angeordnet wird oder daß an bestimmten Stellen der Oberfläche des Trägerchips wenigstens eine Schicht aufgedampft oder aufgetragen wird.

Für eine Untersuchung oder Behandlung von biologischen Zellen ist es vorteilhaft, wenn der Abstand zwischen dem elektrischen oder elektronischen Bauelement und dem (den) im Bereich dessen (deren) Meß- oder Wirkraums angeordneten Vorsprung (Vorsprüngen) an den Durchmesser einer biologischen Zelle angepaßt ist und vorzugsweise größer als 4 um und kleiner als 55 um ist. Dadurch kann sich eine Zelle zwischen dem das elektrische oder elektronische Bauelement aufweisenden Überstand und dem Vorsprung unmittelbar an dem Bauelement anlagern, während Partikel, deren Abmessungen größer sind als der Zelldurchmesser von dem Bauelement ferngehalten werden.

Besonders vorteilhaft ist, wenn in die Substratplatte wenigstens zwei Trägerchips eingesetzt sind, wenn einer der Trägerchips zumindest ein als Strahlungs-Emitter ausgebildetes Bauelement und der andere Trägerchip zumindest ein als Empfänger ausgebildetes, dem Strahlungs-Emitter zugeordnetes Bauelement hat und wenn zwischen den Strahlungs-Emitter und dem Empfänger eine Meßstrecke angeordnet ist. Mit einer solchen Chip-Anordnung kann beispielsweise eine Streulicht- oder Durchlichtmessung durchgeführt werden. Dabei können die beiden Trägerchips gegebenenfalls gleichzeitig auch einen mechanischen Filter bilden, so daß nur Partikel bis zu einer bestimmten, durch den Abstand der Trägerchips vorgegebenen Größe in die Meßstrecke gelangen können.

Vorteilhaft ist, wenn die Substratplatte aus einem elastischen Material besteht, beispielsweise aus amorphem Silizium. Die Substratplatte kann dann in Erstreckungsrichtung mit einer Zugoder Druckkraft beaufschlagt werden, um den Abstand zwischen den das elektrische oder elektronische Bauelement aufweisenden Überstand des Trägerchips und einem mit diesem einen mechanischen Filter bildenden Vorsprung der Substratplatte zu verändern. Dadurch kann die Filtercharakteristik des mechanischen Filters auf einfache Weise an die Größe der zu untersuchenden oder zu behandelnden Partikel angepaßt werden. Gegebenenfalls kann die Substratplatte auch als biegbare Folie ausgebildet sein. Die Chip-Anordnung ist dann noch besser handhabbar.

Die Chip-Anordnung kann noch kostengünstiger hergestellt werden, wenn die Substratplatte wenigstens vier in einer Ebene angeordnete Plattenteile aufweist, wenn zueinander benachbarte Plattenteile jeweils an ihren einander zugewandten Randbereichen vorzugsweise durch eine Klebung miteinander verbunden sind, und wenn der Durchbruch durch einen zwischen den Plattenteilen befindlichen Freiraum gebildet ist. Dadurch kann ein teueres Bohren des Durchbruchs, beispielsweise mittels Ultraschall oder eines Laserstrahls entfallen. Auch kann an dem den Durchbruch begrenzenden Rand der Substratplatte ein Grat, wie er beispielsweise beim Laserbohren auftreten kann, vermieden werden. Die einzelnen Plattenteile weisen vorzugsweise jeweils gerade Ränder auf und können beispielsweise durch Trennschleifen oder Sägen zugeschnitten werden.

Besonders vorteilhaft ist, wenn wenigstens zwei erste Plattenteile jeweils zumindest einen geraden Randbereich aufweisen, mit denen sie parallel zueinander und einander zugewandt angeordnet sind, und wenn zwischen den ersten Plattenteilen in Erstreckungsrichtung der geraden Randbereiche durch den Durchbruch voneinander beabstandet zumindest zwei zweite Plattenteile angeordnet sind, die jeweils an ihren parallel zueinander verlaufenden Rändern mit den geraden Randbereichen der ersten Plattenteile insbesondere durch eine Klebung verbunden sind. Die aneinander anliegenden ersten und zweiten Plattenteile können dann vor dem Anbringen der Klebung in Richtung ihrer geraden Randbereiche gegeneinander verschoben werden, wodurch die Länge des in der Substratplatte befindlichen Durchbruchs auf einfache Weise verändert und an die Abmessungen des darin einzusetzenden Trägerchips angepaßt werden kann.

Zweckmäßigerweise ist der quer zur Trägerchip-Erstreckungsebene angeordnete stirnseitige Endbereich des Trägerchips zumindest im Bereich des das Bauelement aufweisenden Überstands mit einer Isolationsschicht abgedeckt. Dadurch wird bei einem Halbleiter-Trägerchip ein Kurzschluß zwischen dem Substrat des Trägerchips und einem in den Trägerchip integrierten elektronischen Bauelement, beispielsweise einem Sensor, vermieden, wenn der das elektronische Bauelement aufweisende Überstand des Trägerchips mit einem elektrisch leitfähigen Medium, zum Beispiel einem Nährmedium für biologische Zellen, in Verbindung gebracht wird.

An der den Anschlußkontakten zugewandten Rückseite der Substratplatte kann eine Leiterplatte angeordnet sein, die mit den Anschlußkontakten verbundene oder verbindbare Anschlußstellen aufweist. Dadurch ergibt sich ein besonders kompakter Aufbau. Die Leiterplatte kann beispielsweise eine Auswertevorrichtung und/oder eine Steuereinrichtung und/oder eine Stromversorgung für die Chip-Anordnung aufweisen. Diese ist an der Rückseite der Substratplatte vor Berührung mit einem zu untersuchenden Medium geschützt.

Nachfolgend ist ein Ausführungsbeispiel der Erfindung anhand der Zeichnung näher erläutert. Es zeigen:

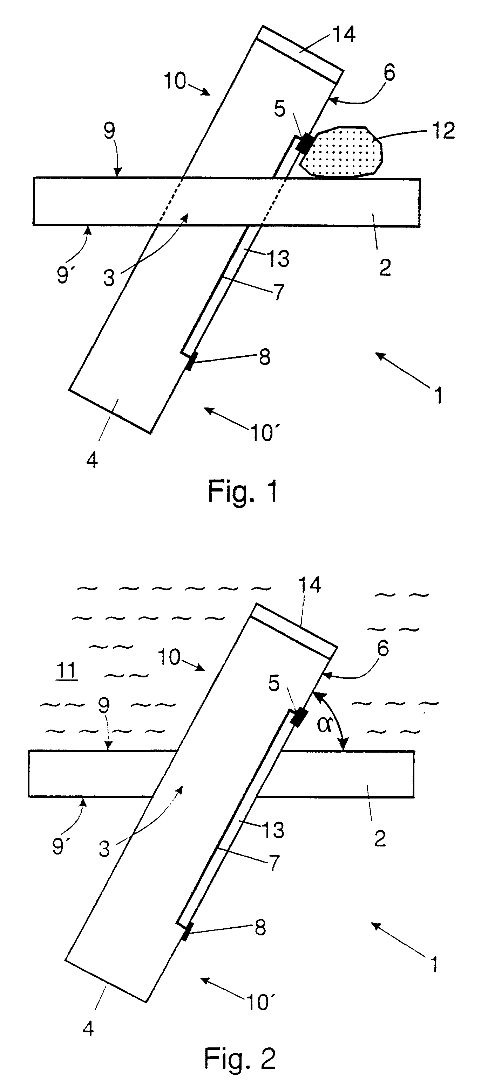

- Fig. 1: eine Seitenansicht einer Chip-Anordnung, bei der die Erstreckungsebenen der Substratplatte und des Trägerchips gegeneinander geneigt sind und
- Fig. 2: einen Querschnitt durch die in Fig. 1 gezeigte Chip-Anordnung.

Eine im ganzen mit 1 bezeichnete Chip-Anordnung weist eine Substratplatte 2 mit einem Durchbruch 3 auf, in den ein Trägerchip 4 eingesetzt ist. Die Substratplatte 2 kann beispielsweise aus Glas oder einem Halbleitermaterial bestehen. Der Trägerchip 4 weist mehrere als Sensoren ausgebildete elektronische Bauelemente 5 auf, die an einer flachseitigen Oberfläche 6 des Trägerchips 4 mit Methoden der Halbleitertechnik in den Trägerchip 4 integriert sind. Die einzelnen Bauelemente 5 sind jeweils mit einer an der Oberfläche 6 des Trägerchips 4 oder im wesentlichen parallel dazu verlaufenden Leiterbahn 7 mit einem Anschlußkontakt 8 verbunden, an dem eine Auswerte- und Steuereinrichtung anschließbar ist. Der Trägerchip 4 ist so in den Durchbruch 3 der Substratplatte 2 eingesetzt, daß er mit seinen Enden die einander abgewandten flachseitigen Oberflächen 9, 9' der Substratplatte 2 überragt und dadurch Überstände 10, 10' bildet, die an den flachseitigen Oberflächen 9, 9' der Substratplatte 2 vorstehen. Dabei sind die Bauelemente 5 an dem einen Überstand 10 und die diesen jeweils zugeordneten elektrischen Anschlußkontakte 8 an dem anderen Überstand 10' angeordnet.

Die Leiterbahnen 7, welche die Bauelemente 5 mit den Anschlußkontakten 8 verbinden, durchsetzen den Durchbruch 3 der Substratplatte 2. Der Trägerchip 4 ist mit dem den Durchbruch 3 der Substratplatte 2 begrenzenden Rand der Substratplatte 2 verklebt, wobei der zwischen diesem Rand und dem Trägerchip 4 befindliche Klebstoff den Trägerchip 4 gegen die Substratplatte 2 abdichtet. Somit sind die an der Rückseite der Substratplatte 2 befindlichen Anschlußkontakte 8 gut gegen ein an der den Bauelementen 5 zugewandten Vorderseite der Substratplatte befindliches, mit den als Sensoren ausgebildeten Bauelementen 5 zu untersuchendes Medium, das beispielsweise ein Nährmedium 11 mit darin befindlichen biologischen Zellen sein kann, abgeschirmt. Eine Korrosion an den Anschlußkontakten 8 durch in dem Nährmedium 11 enthaltene Bestandteile, wie beispielsweise Salze oder Ionen, wird dadurch zuverlässig vermieden. Da die Leiterbahnen 7 den Durchbruch 3 der Substratplatte 2 durchsetzen, brauchen bei der Herstellung der Chip-Anordnung zum Verbinden der Bauelemente 5 mit den elektrischen Anschlußkontakten 8 keine Durchkontaktierungen in die Substratplatte 2 eingebracht werden. Die Chip-Anordnung 1 ist dadurch einfach und kostengünstig herstellbar.

Die das Bauelement 5 und den Anschlußkontakt 8 aufweisende Chip-Oberfläche 6 ist in einer rechtwinklig zu den flachseitigen Oberflächen 9, 9' der Substratplatte 2 verlaufenden Ebene schräg zu diesen Oberflächen 9, 9' angeordnet und schließt mit diesen einen spitzen Winkel α ein. Das Bauelement 5 ist an der flachseitigen Oberfläche 6 des Trägerchips 4 mit Abstand zu den Rändern dieser Oberfläche 6 angeordnet. Der Zutrittsbereich zu dem Bauelement 5 ist also durch den Trägerchip 4 und die Substratplatte 2 begrenzt, wobei der Öffnungswinkel α des Zutrittsbereichs so gewählt ist, daß Partikel, die eine vorgegebene Größe überschreiten von dem Bauelement 5 ferngehalten werden.

Die Chip-Anordnung 1 kann beispielsweise dazu verwendet werden, um mit dem Trägerchip 4 einen Schweißtropfen 12 an der Hautoberfläche einer zu untersuchenden Person abzustreifen. Das Bauelement 5 kann beispielsweise ein Glucose-Sensor sein. Die Chip-Anordnung 1 ermöglicht dann auf einfache Weise eine nichtinvasive Messung des Glucosegehalts, was insbesondere für Diabetiker, die mehrmals am Tag ihren Glucosegehalt bestimmen müssen, vorteilhaft ist. Das Bauelement 5 kann aber auch ein Lactat-Sensor sein, der beispielsweise zur Messung der Lactatkonzentration im Schweißtrop-fens eines Sportlers verwendet werden kann. Dadurch kann auf einfache Weise die Kondition eines Sportlers überprüft werden.

Das Bauelement 5 kann auch ein Sauerstoffsensor auf Clark-Zellenbasis, ein Stickstoffsensor, ein Sensor zur Messung einer Ionenkonzentration oder ein Thermoelement sein. Es kann aber auch ein Bauelement 5 verwendet werden, das ein elektrisches oder elektromagnetisches Feld aussendet, mit dem eine an der Substratplatte 2 befindliche Zelle beeinflußt oder stimuliert werden kann.

Die Leiterbahnen 7 sind mit einer elektrisch isolierenden Dünnfilm-Passivierungsschicht 13 abgedeckt, die beispielsweise aus Siliziumoxid bestehen kann. Durch die Passivierungsschicht 13 sind die Leiterbahnen 7 gegen das Nährmedium 11 elektrisch gut isoliert. Außerdem wird durch die Passivierungsschicht 13 eine Korrosion an den Leiterbahnen 7 durch in dem Nährmedium 11 enthaltene Salze oder Ionen verhindert.

Der quer zur Chip-Erstreckungsebene angeordnete stirnseitige Endbereich des Trägerchips 4 ist im Bereich des das Bauelement 5 aufweisenden Überstands 10 mit einer Isolationsschicht 14 abgedeckt. Dadurch wird ein Stromfluß von dem elektrischen Bauelement 5 über das elektrisch leitfähige Nährmedium 11 in das Substrat der Substratplatte 2 verhindert.

Insgesamt ergibt sich somit eine Chip-Anordnung 1, die eine Substratplatte 2 hat, die einen Durchbruch 3 aufweist, in den ein Trägerchip 4 eingesetzt ist, der ein elektrisches oder elektronisches Bauelement 5 aufweist. In den Trägerchip 4 ist wenigstens eine Leiterbahn 7 integriert, die das Bauelement 5 mit dem elektrischen Anschlußkontakt 8 verbindet. Der Trägerchip 4 ist derart in den Durchbruch 3 eingesetzt, daß er mit seinen Enden die einander abgewandten flachseitigen Oberflächen 9, 9' der Substratplatte 2 überragt und dadurch Überstände 10, 10' bildet. Dabei ist an dem die eine Oberfläche 9 überragenden Überstand 10 das Bauelement und an dem die andere Oberfläche 9' überragenden Überstand 10' der Anschlußkontakt 8 angeordnet und die das Bauelement 5 und den Anschlußkontakt 8 miteinander verbindende Leiterbahn 7 durchsetzt den Durchbruch 3. Zwischen der Substratplatte 2 und dem Trägerchip 4 ist eine Abdichtung angeordnet.

## Patentansprüche

1. Chip-Anordnung (1) mit einer Substratplatte (2), die wenigstens einen Durchbruch (3) aufweist, in den ein Trägerchip (4) derart eingesetzt ist, daß er mit seinen Enden die einander abgewandten flachseitigen Oberflächen (9, 9') der Substratplatte (2) überragt und dadurch Überstände (10, 10') bildet, wobei an dem die eine Oberfläche (9) überragenden Überstand (10) wenigstens ein elektrisches oder elektronisches Bauelement und an dem die andere Oberfläche (9') überragenden Überstand (10') zumindest ein elektrischer Anschlußkontakt (8) angeordnet sind, wobei der Trägerchip (4) an seiner Trägerchip-Oberfläche wenigstens eine den Durchbruch (3) der Substratplatte (2) durchsetzende integrierte Leiterbahn (7) aufweist, die das Bauelement (5) und den Anschlußkontakt (8) miteinander verbindet, und wobei zwischen der Substratplatte (2) und dem Trägerchip (4) eine Abdichtung vorgesehen ist, **dadurch gekennzeichnet, daß** die das elektrische oder elektronische Bauelement (5) aufweisende Trägerchip-Oberfläche schräg zur flachseitigen Oberfläche (9, 9') der Substratplatte (2) angeordnet ist, so daß das Bauelement (5) in einem spitzen Winkel eingeschlossen ist.

2. Chip-Anordnung nach Anspruch 1, **dadurch gekennzeichnet, daß** die Leiterbahn(en) (7) zumindest im Bereich des das elektrische oder elektronische Bauelement (5) aufweisenden Überstandes (10) mit einer elektrisch isolierenden Dünnfilm-Passivierungsschicht abgedeckt ist (sind).

3. Chip-Anordnung nach Anspruch 1 oder 2, **dadurch gekennzeichnet, daß** sich der Querschnitt des das elektrische oder elektronische Bauelement (5) aufweisenden Überstandes (10) ausgehend von der Oberfläche (9) der Substratplatte (2) zu der am weitesten vorstehenden Stelle des Überstandes (10) verjüngt.

4. Chip-Anordnung nach einem der Ansprüche 1 bis 3, **dadurch gekennzeichnet, daß** der Trägerchip (4) bei der Montage der Chip-Anordnung (1) in wenigstens zwei unterschiedlichen Lagen in den Durchbruch (3) der Substratplatte (2) einsetzbar ist, daß in einer dieser Lagen wenigstens ein elektrisches oder elektronisches Bauelement (5) an einem eine flachseitige Oberfläche der Substratplatte (9) überragenden Überstand (10) des Trägerchips (4) und der (die) diesem (diesen) Bauelement(en) (5) zugeordnete(n) Anschlußkontakt(e) (8) an dem die andere flachseitige Oberfläche (9') der Substratplatte überragenden Überstand (10') angeordnet ist, und daß in der anderen Lage des Trägerchips (4) das (die) Bauelement(e) (5) und der (die) Anschlußkontakt(e) (8) an demselben, eine flachseitige Oberfläche der Substratplatte (9, 9') überragenden Überstand (10, 10') des Trägerchips (4) angeordnet sind.

5. Chip-Anordnung nach einem der Ansprüche 1 bis 4, **dadurch gekennzeichnet, daß** der Trägerchip (4) bei der Montage der Chip-Anordnung (1) in wenigstens zwei unterschiedlichen Lagen in den Durchbruch (3) der Substratplatte (2) einsetzbar ist, daß der Trägerchip (4) wenigstens zwei elektrische oder elektronische Bauelemente (5) aufweist, die jeweils mittels wenigstens einer Leiterbahn (7) mit zumindest einem ihnen jeweils zugeordneten elektrischen Anschlußkontakt (8) verbunden sind, und daß je nach gewählter Lage des Trägerchips (4) jeweils wenigstens eines dieser Bauelemente (5) an einem eine flachseitige Oberfläche der Substratplatte (9) überragenden Überstand (10) des Trägerchips (4) und der (die) diesem (diesen) Bauelement(en) (5) zugeordnete(n) Anschlußkontakt(e) (8) an dem die andere flachseitige Oberfläche (9') der Substratplatte überragenden Überstand (10') angeordnet ist.

6. Chip-Anordnung nach einem der Ansprüche 1 bis 5, **dadurch gekennzeichnet, daß** die Substratplatte im Bereich des Meßoder Wirkraumes des elektrischen oder elektronischen Bauelements (5) wenigstens einen Vorsprung aufweist, der zusammen mit dem das Bauelement aufweisenden Überstand (10) einen mechanischen Filter bildet.

7. Chip-Anordnung nach Anspruch 6, **dadurch gekennzeichnet, daß** die das elektrische oder elektronische Bauelement (5) aufweisende Trägerchip-Oberfläche und die dieser zugewandten Oberfläche des im Bereich des Meß- oder Wirkraums des Bauelements (5) angeordneten Vorsprungs in der Oberflächenebene der Substratplatte (2) trichterförmig schräg zueinander verlaufen.

8. Chip-Anordnung nach einem der Ansprüche 1 bis 7, **dadurch gekennzeichnet, daß** an dem Trägerchip (4) ein Körper anliegt, der das elektrische oder elektronische Bauelement (5) überdeckt, daß als Abstandshalter an dem Trägerchip (4) mindestens ein seitlich über die Oberflächenebene des Bauelements (5) vorstehender, an dem Körper anliegender Bereich und/oder an dem Körper ein seitlich über den das Bauelement (5) überdeckenden Oberflächenbereich vorstehender, an dem Trägerchip (4) anliegender Bereich angeordnet ist, derart, daß zwischen dem Bauelement (5) und dem Körper ein den Zugang zu dem Bauelement bildender Freiraum oder Spalt angeordnet ist.

9. Chip-Anordnung nach einem der Ansprüche 1 bis 8, **dadurch gekennzeichnet, daß** in die Substratplatte (2) wenigstens zwei Trägerchips (4) eingesetzt sind, daß einer der Trägerchips (4) zumindest ein als Strahlungs-Emitter ausgebildetes Bauelement (5) und der andere Trägerchip (4) zumindest ein als Empfänger ausgebildetes, dem Strahlungs-Emitter zugeordnetes Bauelement (5) hat und daß zwischen dem Strahlungs-Emitter und dem Empfänger eine Meßstrecke angeordnet ist.

10. Chip-Anordnung nach einem der Ansprüche 1 bis 9, **dadurch gekennzeichnet, daß** das elektrische oder elektronische Bauelement (5) mit einer in den Trägerchip (4) integrierten Auswerte- und oder Steuereinheit verbunden ist.

## Claims

1. A chip arrangement (1) with a substrate plate (2) which has at least one opening (3) in which a carrier chip (4) is placed in such a way that its ends project beyond the opposing flat-sided surfaces (9, 9') of the substrate plate (2), thereby forming projections (10, 10'), wherein at least one electrical or electronic component is disposed on the projection (10) which projects beyond the one surface (9) and at least one electrical connection contact (8) is disposed on the projection which projects beyond the other surface (9'), wherein the carrier chip (4) has at least one integrated conductor track (7) on its carrier chip surface which penetrates the opening (3) of the substrate plate (2) and which connects the component (5) and the connection contact (8), and wherein a seal is provided between the substrate plate (2) and the carrier chip (4), **characterised in that** the carrier chip surface which holds the electrical or electronic component (5) is disposed obliquely to the flat-sided surface (9, 9') of the substrate plate (2) so that the component (5) is enclosed in an acute angle.

2. A chip arrangement according to claim 1, **characterised in that** the conductor track(s) (7) is/are covered with an electrically-insulating thin film passivation layer, at least in the area of the projection (10) which holds the electrical or electronic component (5).

3. A chip arrangement according to claim 1 or 2, **characterised in that** the cross-section of the projection (10) which holds the electrical or electronic component (5) tapers from the surface (9) of the substrate plate (2) to the furthest-projecting part of the projection (10).

4. A chip arrangement according to one of claims 1 to 3, **characterised in that** the carrier chip (4) can be placed in the opening (3) of the substrate plate (2) in at least two different positions during assembly of the chip arrangement (1), that in one of these positions, at least one electrical or electronic component (5) is disposed on a projection (10) of the carrier chip (4) which projects over a flat-sided surface of the substrate plate (9) and the connection contact(s) (8) assigned to this (these) component(s) (5) are arranged on the other flat-sided surface (9') of the projection (10') which projects beyond the substrate plate, and that in the other position of the carrier chip (4) the component(s) (5) and the connection contact(s) are disposed on the same projection (10, 10') of the carrier chip (4) which projects over a flat-sided surface of the substrate plate (9, 9').

5. A chip arrangement according to one of claims 1 to 4, **characterised in that** the carrier chip (4) can be placed in the opening (3) of the substrate plate (2) in at least two different places during assembly of the chip assembly (1), that the carrier chip (4) has at least two electrical or electronic components (5) which are each connected to at least one assigned electrical connection contact (8) via at least one conductor track (7), and that according to the selected position of the carrier chip (4), in each case at least one of these components (5) is disposed on a projection (10) of the carrier chip (4) which projects over a flat-sided surface of the substrate plate (9) and the connection contact(s) (8) assigned to this (these) component(s) (5) is (are) disposed on the projection (10') which projects over the other flat-sided surface (9') of the substrate plate.

6. A chip arrangement according to one of claims 1 to 5, **characterised in that** the substrate plate has at least one projection in the area of the measurement or operating space of the electrical or electronic component (5) which, together with the projection (10) which holds the component, forms a mechanical filter.

7. A chip arrangement according to claim 6, **characterised in that** the carrier chip surface which holds the electrical or electronic component (5) and the surface of the projection disposed in the area of the measurement or operating space of the component (5) which faces this extend towards one another in the shape of a funnel in the surface plane of the substrate plate (2).

8. A chip arrangement according to one of claims 1 to 7, **characterised in that** a body abuts against the carrier chip (4) which covers the electrical or electronic component (5) disposed on the carrier chip (4), that as a distance piece, at least one area is provided on the carrier chip (4), abutting against the body and projecting laterally over the surface plane of the component (5), and/or an area is disposed on the body, which projects laterally over the surface area which covers the component (5) and which abuts the carrier chip (4) in such a way that a free space or gap is disposed between the component (5) and the body which provides access to the component.

9. A chip arrangement according to one of claims 1 to 8, **characterised in that** at least two carrier chips (4) are placed in the substrate plate (2), that one of the carrier chips (4) has at least one component (5) configured as a radiation emitter and the other carrier chip (4) has at least one component (5) configured as a receiver which is assigned to the radiation emitter, and that a measurement distance is arranged between the radiation emitter and the receiver.

10. A chip arrangement according to one of claims 1 to 9, **characterised in that** the electrical or electronic component (5) is connected to an evaluation and/or control unit integrated into the carrier chip (4).

## Revendications

1. Agencement de puce (1) avec une plaque de substrat (2), qui comporte au moins une ouverture (3), dans laquelle une puce porteuse (4) est insérée de telle sorte qu'elle dépasse avec ses extrémités les surfaces (9, 9') planes opposées l'une à l'autre de la plaque de substrat (2) et de ce fait forme des parties saillantes (10, 10'), la partie saillante (10) dépassant de l'une (9) des surfaces comportant au moins un composant électrique ou électronique et la partie saillante (10') dépassant de l'autre surface (9') comportant au moins un contact de raccordement électrique (8), la puce porteuse (4) comportant, sur sa surface de puce porteuse, au moins une piste de circuit imprimé (7) intégrée, traversant l'ouverture (3) de la plaque de substrat (2), et reliant ensemble le composant (5) et le contact de raccordement (8), et un joint d'étanchéité étant prévu entre la plaque de substrat (2) et la puce porteuse (4), **caractérisée en ce que** la surface de puce porteuse, comportant le composant électrique ou électronique (5) est disposée de façon oblique à la surface plane (9, 9') de la plaque de substrat (2) de telle façon que le composant (5) soit enfermé dans un angle aigu.

2. Agencement de puce selon la revendication 1, **caractérisée en ce que** la/les piste(s) de circuit imprimé (7) est/sont recouverte(s), au moins dans la zone de la partie saillante (10) comportant le composant électrique ou électronique (5), avec une mince couche de passivation, isolante en termes d'électricité.

3. Agencement de puce selon la revendication 1 ou 2, **caractérisée en ce que** la section de la partie saillante (10) comportant le composant électrique ou électronique (5) se rétrécit à partir de la surface (9) de la plaque de substrat (2) jusqu'à la position dépassant le plus de la partie saillante (10).

4. Agencement de puce selon l'une quelconque des revendications 1 à 3, **caractérisée en ce que** la puce porteuse (4) peut être placée lors du montage de la agencement de puce (1) au moins dans deux positions différentes au sein de l'ouverture (3) de la plaque de substrat (2), que dans l'une de ces positions, au moins. un composant électrique ou électronique (5) est placé sur une partie saillante (10) de la puce porteuse (4), dépassant d'une surface plane de la plaque de substrat (9) et le (les) contact(s) de raccordement (8) attribué (s) à ce (ces) composant(s) (5) est (sont) placé(s) sur la partie saillante (10') dépassant de l'autre surface (9') plane de la plaque de substrat et **en ce que**, dans l'autre position de la puce porteuse (4) le (les) composant(s) (5) et le (les) contact(s) de raccordement (8) sont placés sur la même partie saillante (10, 10') de la puce porteuse (4) dépassant d'une surface plane de la plaque de substrat (9, 9').

5. Agencement de puce selon l'une quelconque des revendications 1 à 4, **caractérisée en ce que** la puce porteuse (4) peut être placée lors du montage de la agencement de puce (1) au moins dans deux positions différentes dans l'ouverture (3) de la plaque de substrat (2), que la puce porteuse (4) comporte au moins deux composants électriques ou électroniques (5), qui sont respectivement reliés, grâce à au moins une piste de circuit imprimé (7), avec au moins un contact de raccordement électrique (8) qui lui est respectivement attribué et **en ce qu'**en fonction de la position sélectionnée de la puce porteuse (4), au moins un de ces composants (5) est, à chaque fois, placé sur la partie saillante (10) de la puce porteuse (4) dépassant de l'une des surfaces planes de la plaque de substrat (9) et le (les) contact(s) de raccordement électrique (8) attribué(s) à ce (ces) composant(s) (5) sont placés sur la partie saillante (10') dépassant de l'autre surface plane de la plaque de substrat (9').

6. Agencement de puce selon l'une quelconque des revendications 1 à 5, **caractérisée en ce que** la plaque de substrat comporte dans la zone de mesure ou du rayon d'action du composant électrique ou électronique (5) au moins une saillie, qui forme, avec la partie saillante (10) du composant, un filtre mécanique.

7. Agencement de puce selon la revendication 6, **caractérisée en ce que** la surface de la puce porteuse comportant le composant électrique ou électronique (5) et la surface de la saillie, située dans la zone de mesure ou de rayon d'action du composant (5) et orientée vers. la première surface s'étendent dans le. plan superficiel de la plaque de substrat (2) obliquement sous la forme d'un entonnoir.

8. Agencement de puce selon l'une quelconque des revendications 1 à 7, **caractérisée en ce qu'**un corps, qui recouvre le. composant électrique ou électronique (5), est en contact avec la puce porteuse (4), qu'au moins une zone en contact avec la puce porteuse (4) et dépassant de la surface latérale du plan superficiel du composant (5) est placée comme écarteur au niveau de la puce porteuse (4) et / ou qu'une zone en contact avec la puce porteuse (4) et dépassant latéralement de la zone de surface recouvrant le composant (5) est placée au niveau du corps de manière à ce qu'entre le composant (5) et le corps, un espace libre ou une fente permettant un accès au composant soit laissé.

9. Agencement de puce selon l'une quelconque des revendications 1 à 8, **caractérisée en ce qu'**au moins deux puces porteuses (4) sont implantées dans la plaque de substrat (2), **en ce qu'**une des puces porteuses (4) a au moins un composant (5) configuré comme un émetteur de rayonnement et **en ce que** l'autre puce porteuse (4) a au moins un composant (5) configuré comme un récepteur et affecté à l'émetteur de rayonnement et **en ce qu'**une section de mesure est agencée entre l'émetteur et le récepteur.

10. Agencement de puce selon l'une quelconque des revendications 1 à 9, **caractérisée en ce que** le composant électrique ou électronique (5) est relié à une unité de commande ou/et d'évaluation intégrée dans la puce porteuse (4).
